# EUROPEAN PATENT SPECIFICATION

(11) **EP 3 228 242 B1**
(45) Date of publication and mention of the grant of the patent: **24.06.2020**
(21) Application number: 17150094.5
(22) Date of filing: 03.01.2017
(51) Int. Cl.: A61B 5/00, B41J 2/01, D06M 15/15, B05D 3/02, B05D 1/38, B05D 3/10, B41J 3/407, B41J 11/00, B05D 1/26, B05D 1/36, B05D 5/12, H01B 1/12

(54) **METHOD FOR PRODUCING A CONDUCTIVE POLYMER CONDUCTOR**
VERFAHREN ZUR HERSTELLUNG EINES LEITFÄHIGES POLYMERLEITERS
MÉTHODE DE FABRICATION D'UN POLYMÈRE CONDUCTEUR SUR UN SUPPORT

(30) Priority: 05.04.2016 JP 2016076094
(43) Date of publication of application: 11.10.2017
(73) Proprietor: AI Silk Corporation, Miyagi 980-8579 (JP)
(72) Inventor: OKANO, Hideo, Miyagi, Miyagi 980-8579 (JP); WATANABE, Satoshi, Miyagi, Miyagi 980-8579 (JP)
(74) Representative: TBK

(56) References cited:
- WO-A1-94/25967
- WO-A1-2006/070186
- WO-A1-2013/096356
- WO-A1-2014/157550
- WO-A1-2015/037481
- WO-A1-2016/031872
- JP-A- H05 283 304
- JP-A- 2000 052 644
- JP-A- 2009 072 729
- KLAUS OPWIS ET AL: "Oxidative in situ deposition of conductive PEDOT:PTSA on textile substrates and their application as textile heating element", SYNTHETIC METALS, vol. 162, no. 21-22, 1 December 2012 (2012-12-01), pages 1912-1918, XP055360629, CH ISSN: 0379-6779, DOI: 10.1016/j.synthmet.2012.08.007
- DATABASE WPI Week 200704 2007 Thomson Scientific, London, GB; AN 2007-026633 XP002772156, -& CN 1 749 476 A (UNIV TIANJIN POLYTECHNIC) 22 March 2006 (2006-03-22)
- Anonymous: "45th NEPCON JAPAN Exhibitor Directory", , 13 January 2016 (2016-01-13), XP55386280, Retrieved from the Internet: URL:https://www.r-expo.jp/inw2016/exhiSear ch/INW/en/search_detail.php?id=1588 [retrieved on 2017-06-28]
- DATABASE WPI Week 201458 2014 Thomson Scientific, London, GB; AN 2014-P57821 XP002772157, -& KR 2014 0095199 A (UNIV YONSEI IND ACADEMIC COOP FOUND) 1 August 2014 (2014-08-01)
- DATABASE WPI Week 201477 2014 Thomson Scientific, London, GB; AN 2014-U99602 XP002772158, -& CN 104 010 446 A (UNIV XIAN POLYTECHNIC) 27 August 2014 (2014-08-27)

## Description

### Technical Field

The invention relates to a method for producing a conductive polymer conductor in which a conductive polymer is used.

### Background Art

Conductive polymer fibers have been recently known in which a conductive polymer such as PEDOT-PSS {poly(3,4-ethylenedioxythiophene)-poly(styrene sulfonic acid)} is adhered to base material fibers such as silk (for example, see Patent Literature 1 and Patent Literature 2). The conductive polymer fibers have electrical conductivity, hydrophilicity, tensile strength and water-resistant strength, and therefore can be used particularly as a material for a biological electrode. However, the conductive polymer fibers described in Patent Literature 1 is prepared by adhering the conductive polymer to the base material fibers by an electrochemical method, and have involved problems of lack in simplicity in comparison with a chemical method, and necessity of using a conductive material for the base material fibers. When the conductive polymer is adhered to the base material by the chemical method, furthermore by applying a printing method, the conductive polymer fibers can be simply produced, as described in Patent Literature 2, and simultaneously the conductive polymer can be adhered thereto in a free shape which is preferable.

### Citation List

### Patent Literature

Patent Patent Literature 1: JP 2015-77414 A and Patent Literature 2: WO2016/031872 A1.

### Summary of Invention

### Technical Problem

However, if an attempt is made on printing a monomer of a conductive polymer on a base material to allow a chemical polymerization reaction thereon, bleeding is eventually caused, resulting in a problem of difficulty in adhering the conductive polymer thereto in an objective shape with high accuracy.

The invention has been made based on such a problem, and contemplated for providing a method for producing a conductive polymer conductor easily adhered to the base material with high accuracy.

### Solution to Problem

A method for producing a conductive polymer conductor according to claim 1 is provided for producing the conductive polymer conductor in which a conductive polymer is adhered to a base material, and the method including:
a raw material application step of applying, to a base material, a raw material solution containing a monomer of a conductive polymer after heating the base material or while heating the base material; and
a producing solution application step of applying, to the base material being in a heated state, a producing solution containing an oxidizing agent for promoting polymerization of the monomer, a dopant for developing electrical conductivity in the conductive polymer, and a viscosity improver for improving viscosity after applying the raw material solution to the base material.

A device for producing a conductive polymer conductor is disclosed for producing a conductive polymer conductor in which the conductive polymer is adhered to a base material, and the device comprising:
a heating means for heating the base material;
a raw material application means for applying, to the base material, a raw material solution containing a monomer of the conductive polymer;
a producing solution application means for applying , to the base material, a producing solution containing an oxidizing agent for promoting polymerization of the monomer, a dopant for developing electrical conductivity in the conductive polymer, and a viscosity improver for improving viscosity.

### Advantageous Effects of Invention

A method for producing a conductive polymer conductor according to claim 1 is contemplated in such a manner that a raw material solution containing a monomer of a conductive polymer is applied to a base material being in a heated state, after heating the base material or while heating the base material, and a producing solution containing an oxidizing agent and a dopant is subsequently applied thereto, and therefore a chemical polymerization reaction can be immediately performed, and bleeding can be minimized. Moreover, a viscosity improver is added to the producing solution, and therefore spreading of the producing solution can be suppressed upon applying the producing solution to the base material, and the bleeding can be further minimized. Moreover, addition of the viscosity improver simultaneously causes action of reducing a resistance value to a half or less. Accordingly, the conductive polymer can be adhered to the base material into an objective shape with high accuracy and with high conductivity.

Moreover, if unreacted substances in the raw material solution and the producing solution applied to the base material are washed and removed, such operation can prevent the unreacted substances from gradually reacting to cause spreading of the bleeding.

Further, if the raw material solution and the producing solution are applied to the base material by moving the raw material application means for applying the raw material solution thereto, and the producing solution application means for applying the producing solution thereto relative to the base material, such operation can easily adhere the conductive polymer to the base material in the objective shape.

In addition thereto, if the base material is partially heated by moving a position on which the base material is heated in corresponding to positions of the raw material application means and the producing solution application means relative to the base material, such operation can easily control a reaction time.

Furthermore, if a fixing material for fixing the conductive polymer to the base material is applied to the base material, such operation can adhere the conductive polymer thereto, irrespective of a material of the base material.

The disclosed device for producing the conductive polymer conductor is contemplated for having the heating means for heating the base material, the raw material application means for applying the raw material solution to the base material, the producing solution application means for applying the producing solution to the base material, and therefore the method for producing the conductive polymer conductor can be easily realized.

### Brief Description of Drawings

Figure 1 is a drawing showing a configuration of a device for producing a conductive polymer conductor related to one embodiment.
Figure 2 is a flowchart showing steps in a method for producing a conductive polymer conductor related to one embodiment.
Figure 3 is a characteristic drawing showing a relationship among a reaction temperature, a reaction time, and resistance of a conductive polymer conductor obtained.
Figure 4 is a characteristic drawing showing a relationship among a kind of solvent, a pTS concentration and resistance of s conductive polymer conductor obtained.
Figure 5 is a characteristic drawing showing a relationship among a kind of viscosity improver, an amount of addition of the viscosity improver and resistance of a conductive polymer conductor obtained.
Figure 6 is a characteristic drawing showing a relationship among a kind of viscosity improver, an amount of addition of the viscosity improver and a diameter of a spot after reaction.
Figure 7 is a photograph showing a state of a spot after a producing solution is added dropwise to allow reaction.

### Description of Embodiments

The following embodiments are described in detail with reference to drawings.

A method and a device for producing a conductive polymer conductor are disclosed for producing a conductive polymer conductor in which a conductive polymer is adhered to a base material. Specific examples of the conductive polymer to be adhered thereto preferably include poly3,4-ethylenedioxythiophene (hereinafter, described as PEDOT). Specific examples of the base material preferably include a thread-like or sheet-like material. Specific examples of the material which forms the base material includes natural fibers, synthetic fibers, paper or fibers on which sericin is applied. Above all, when PEDOT is used as the conductive polymer, the base material is preferably formed of silk or sericin, because high adhesion can be obtained. When the base material is formed of a material other than silk or sericin, for example, a material prepared by applying sericin to a surface of the base material is preferably used. Sericin is a protein which forms silk. In addition, the conductive polymer conductor can be used for a conductive polymer electrode.

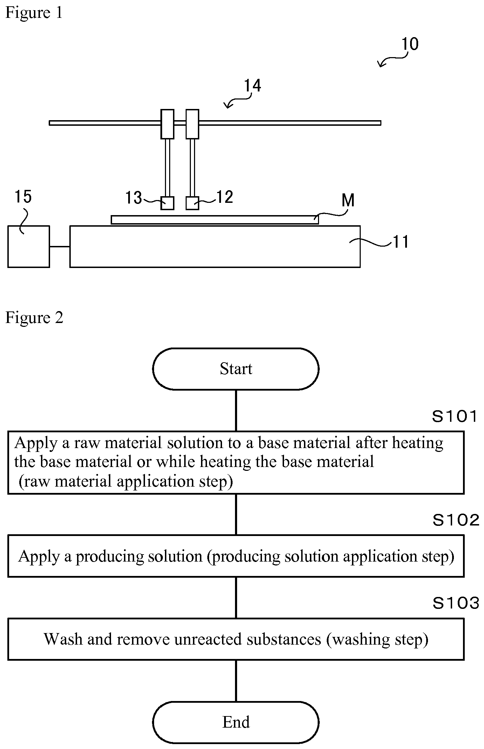

Figure 1 shows a configuration of a device 10 for producing a conductive polymer conductor as related to one embodiment. The device 10 for producing the conductive polymer conductor is equipped with, for example, a heating means 11 for heating the base material M, a raw material application means 12 for applying a raw material solution containing a monomer of the conductive polymer to the base material M, and a producing solution application means 13 for applying, to the base material M, a producing solution containing an oxidizing agent for promoting polymerization of the monomer, a dopant for developing electrical conductivity in the conductive polymer, and a viscosity improver for improving viscosity. As the heating means 11, any kind may be applied, as long as the means can heat the base material M. When a sheet-like base material M is heated, for example, specific examples preferably include a hot plate. The raw material application means 12 has a raw material output unit for outputting the raw material solution, and the producing solution application means 13 has a producing solution output unit for outputting the producing solution, for example.

The raw material application means 12 and the producing solution application means 13 are preferably configured, for example, in such a manner that positions relative to the base material M are movable by an application position moving means 14, because the raw material solution and the producing solution can be easily applied to the base material M in an objective pattern. The application position moving means 14 may be configured in such a manner that the raw material application means 12 and the producing solution application means 13 may be moved each independently, or may be moved in an interlocking manner or simultaneously.

Moreover, the heating means 11 is preferably configured, for example, in such a manner that a position on which the base material M is heated is movable by the heating position control means 15 in corresponding to positions of the raw material application means 12 and the producing solution application means 13 relative to the base material M, and the base material M can be partially heated.

Moreover, the device 10 for producing the conductive polymer conductor preferably has, for example, a washing means for washing and removing unreacted substances in the raw material solution and the producing solution applied to the base material M for the purpose of preventing the unreacted substances from gradually reacting to cause spreading of bleeding. Specific examples of the washing means include a vessel in which a washing solution such as ethanol is put, a sprayer (shower) for spraying the washing solution thereto and a dispenser therefor. The device 10 for producing the conductive polymer conductor may be further equipped with, for example, a fixing material application means for applying a fixing material for fixing the conductive polymer to the base material M. Specific examples of the fixing material application means include a vessel in which a liquefied fixing material is put, a sprayer (shower) for spraying the liquefied fixing material thereto and a dispenser therefor.

Figure 2 shows steps in a method for producing a conductive polymer conductor related to one embodiment. In the method for producing the conductive polymer conductor, for example, the conductor can be produced by using the device 10 for producing the conductive polymer conductor shown in Figure 1. First, a raw material solution containing a monomer of a conductive polymer is applied to a base material M by a raw material application means 12 after heating the base material M or while heating the base material M by a heating means 11 (raw material application step: Step S101).

Next, after the raw material solution is applied to the base material M, a producing solution containing an oxidizing agent for promoting polymerization of the monomer, a dopant for developing electrical conductivity in the conductive polymer, and a viscosity improver for improving viscosity is applied to the base material being in a heated state, by a producing solution application means 13 (producing solution application step: Step S102). The producing solution application step may be performed after the raw material application step is ended or may be performed in parallel before the raw material application step is ended.

Specific examples of the oxidizing agent in the producing solution preferably include an iron salt. Specific examples of the dopant preferably include p-toluenesulfonic acid, and if an iron salt of p-toluenesulfonic acid (hereinafter, described as pTS) is used, and the iron salt can also be functioned as the oxidizing agent, and therefore such a case is further preferable. Specific examples of the dopant include acetonitrile and trifluoroacetic acid, in addition thereto. The viscosity improver is provided for suppressing spreading upon applying the producing solution thereto by increasing the viscosity of the producing solution to minimize bleeding of the conductive polymer. As the viscosity improver, a material which causes no reaction in a polymerization reaction of the conductive polymer is preferable, and specific examples preferably include glycerol, ethylene glycol, gelatin or polysaccharide. The producing solution may contain a solvent. Specific examples of the solvent include an organic solvent such as butanol or ethanol.

In the producing solution application step, the base material M is in a heated state for the purpose of immediately causing a chemical polymerization reaction upon applying the producing solution thereto to minimize the bleeding. A temperature of the base material M upon applying the producing solution thereto, or a reaction temperature is preferably adjusted to 60°C or higher and 120°C or lower, further preferably 70°C or higher and 110°C or lower, and still further preferably 80°C or higher and 100°C or lower, for example. The reason is that, at a temperature lower than 60°C, a rate of the chemical polymerization reaction is low, and the bleeding is easily caused, and at a temperature higher than 120°C, a protein of silk or a polymer is liable to break. As the reaction temperature is higher, a reaction time is preferably adjusted to be shorter, for example, preferably 1 second to 60 seconds, and further preferably 5 seconds to 20 seconds.

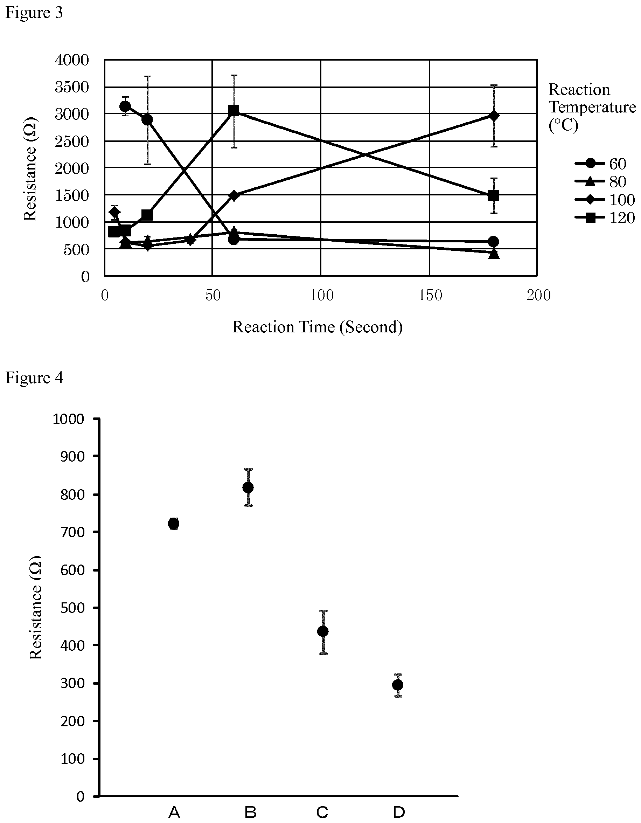

Figure 3 shows the results obtained by examining a relationship among a reaction temperature, a reaction time and resistance of a conductive polymer conductor obtained. In an example shown in Figure 3, resistance between two points apart by 8 mm was measured by placing, on a hot plate, a base material M formed of a ribbon made of silk (Takaramoto Corporation, single satin, width: 12 mm, length: 12 mm), applying and infiltrating, as a raw material solution, 10 µL of PEDOT monomer solution (Heraeus CleviosM-V2) thereto while heating the solution, applying a producing solution containing pTS as an oxidizing agent and a dopant thereto in a 4 mm-wide area by stamping, and holding the resultant material for a predetermined time as a reaction time, and then washing the resultant material by ethanol (Wako Pure Chemical Co., Ltd.).

In addition, the producing solution was prepared by mixing glycerol (Wako Pure Chemical, Co., Ltd.) as a viscosity improver, butanol (Wako Pure Chem.) as a solvent and a 40% butanol solution (Heraeus Clevios C-B 40 V2) of pTS. A ratio of glycerol to butanol in the producing solution was adjusted to 1:1 in a volume ratio, and a pTS concentration in the producing solution was adjusted to be 20 mass%. Moreover, the reaction temperature was changed to 60°C, 80°C, 100°C and 120°C, and the reaction time was changed to 5 seconds, 10 seconds, 20 seconds, 40 seconds, 60 seconds and 180 seconds. Table 1 shows main conditions.

**[Table 1]**

| | | |
|---|---|---|
| Reaction Temperature (°C) | | 60, 80, 100, 120 |
| Reaction Time (Second) | | 5, 10, 20, 40, 60, 180 |
| Common conditions | Viscosity improver: Solvent (Volume Ratio) | Glycerol: Butanol = 1 : 1 |
| | pTS Concentration (Mass%) | 20 |

As shown in Figure 3, at 60°C, the resistance was high when the reaction time was short, and the resistance was reduced in 60 seconds or more. At 80°C, the resistance was consistently low in 10 seconds to 180 seconds. At 100°C, the resistance was low in 10 seconds to 40 seconds, and the resistance increased when the time became longer than the above. At 120°C, the resistance was low up to 20 seconds, but the resistance increased when the time became longer than the above. More specifically, it is found that the temperature of the base material M upon applying the producing solution thereto is preferably adjusted to 60°C or higher and 120°C or lower, and the reaction time is preferably adjusted to be shorter as the temperature is higher, and preferably adjusted to 1 second to 60 seconds.

Figure 4 shows the results obtained by examining a relationship among a kind of solvent, a pTS concentration as an oxidizing agent and a dopant, and resistance of a conductive polymer conductor obtained. In an example shown in Figure 4, the conductive polymer conductor was produced and the resistance was measured in a manner similar to the example shown in Figure 3 except that, as a producing solution, a single material of pTS, glycerol as a viscosity improver, butanol or ethanol as a solvent were mixed, and a reaction temperature was adjusted to 100°C and a reaction time was adjusted to 10 seconds,. The kind of solvent and the pTS concentration in the producing solution of A to D are as shown in Table 2. Moreover, a ratio of glycerol to butanol or methanol was adjusted to 1:1 in glycerol : butanol or methanol in a volume ratio in the producing solution.

**[Table 2]**

| | Solvent | pTS Concentration (Mass%) | Common Conditions | | | |
|---|---|---|---|---|---|---|
| | | | Viscosity Improver | Viscosity Improver: Solvent (Volume Ratio) | Reaction Temperature (°C) | Reaction Time (Second) |
| A | Butanol | 20 | Glycerol | 1 : 1 | 100 | 10 |
| B | Ethanol | 20 | | | | |
| C | Butanol | 40 | | | | |
| D | Ethanol | 40 | | | | |

As shown in Figure 4, no significant difference was found in the resistance depending on the kind of solvent, but the resistance in C and D in which the pTS concentration was increased was about a half in comparison with A and B in which the pTS concentration was low. From the results, it is considered that the pTS concentration is a factor which determines a resistance value and a dopant concentration is preferably increased.

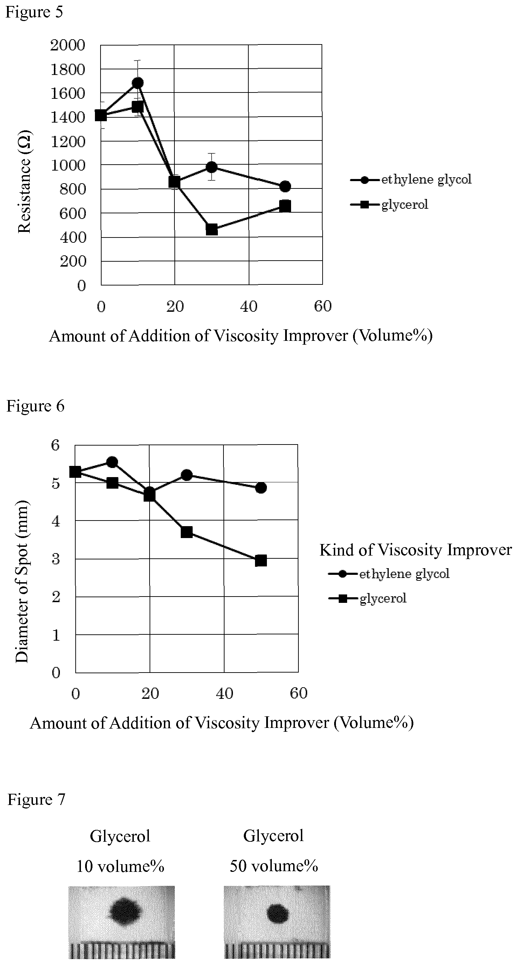

Figure 5 shows the results obtained by examining a relationship among a kind of viscosity improver, an amount of addition of the viscosity improver, and resistance of a conductive polymer conductor obtained. In an example shown in Figure 5, a conductive polymer conductor was produced and resistance was measured in a manner similar to the example shown in Figure 3 except that a kind and an amount of addition of a viscosity improver were changed as shown in Table 3, and a reaction temperature was adjusted to 100°C, and a reaction time was adjusted to 10 seconds. The amount of addition of the viscosity improver is expressed in terms of volume% of the viscosity improver based on a total of the viscosity improver and the solvent.

**[Table 3]**

| | Viscosity Improver | | Common Conditions | | | |
|---|---|---|---|---|---|---|
| | Kind | Amount of Addition (Volume%) | Solvent | pTS Concentration (Mass%) | Reaction Temperature (°C) | Reaction Time (Second) |
| A | Ethylene glycol | 0 to 50 | Butanol | 20 | 100 | 10 |
| B | Glycerol | 0 to 50 | | | | |

Moreover, Figure 6 shows the results obtained by dropwise adding 0.8 µL of the producing solution used in the examples shown in Table 3 and Figure 5, at 100°C, to a silk cloth into which a monomer solution of PEDOT is infiltrated to allow reaction for 10 seconds, and then measuring a diameters of a spot. Further, Figure 7 shows photographs showing states of spots after the producing solution in which an amount of addition of glycerol is 10 volume% and 50 volume% is added dropwise thereto to allow reaction.

As shown in Figure 5, a solution in which the amount of addition of glycerol or ethylene glycol as the viscosity improver was 20 volume% or more showed lower resistance in comparison with a solution in which the amount was 10% or less. Moreover, as shown in Figure 6 and Figure 7, if glycerol or ethylene glycol as the viscosity improver was added thereto, the diameter of the spot was reduced, and the effect was higher in glycerol in comparison with ethylene glycol. More specifically, it is found that, if the viscosity improver is added thereto, bleeding is able to be minimized, and simultaneously the electrical conductivity is able to be improved. In addition, the amount of addition of the viscosity improver is preferably adjusted to 20 volume% to 50 volume%, further preferably 30 volume% to 50 volume%, based on the total of the viscosity improver and the solvent.

In addition, in the raw material application step and the producing solution application step, the raw material solution and the producing solution are preferably applied to the base material M by moving the raw material application means 12 and the producing solution application means 13 relative to the base material M, because such operation can easily apply the raw material solution and the producing solution to the base material M in the objective pattern. Moreover, the base material M is preferably contemplated for being partially heated by moving the position on which the base material M is heated in corresponding to the positions of the raw material application means 12 and the producing solution application means 13 relative to the base material M, because, if the reaction time is elongated, the resistance is eventually increased in several cases.

The base material M is preferably washed with taking a predetermined reaction time after the producing solution application step to wash and remove the unreacted substances in the raw material solution and the producing solution applied (washing step: Step S103) for the purpose of preventing the unreacted substances from gradually reacting to cause spreading of bleeding. In the washing step, for example, the base material M may be washed by putting the base material M in the vessel in which the washing solution such as ethanol is put, or by spraying the washing solution to the base material M by the sprayer (shower), the dispenser.

In addition, for example, the method may include a fixing material application step of applying, to a base material M, a fixing material for fixing a conductive polymer thereto before the raw material solution is applied to the base material M. For example, when PEDOT is used as the conductive polymer, the base material M can be formed of a material having low adhesion with PEDOT by applying sericin having high adhesion with PEDOT as the fixing material. In the fixing material application step, for example, the base material M may be put in a vessel in which a liquefied fixing material is put, and dyed, or may be applied by spraying the liquefied fixing material thereto.

Thus, according to the method for producing the conductive polymer conductor of the present embodiment, the raw material solution containing the monomer of the conductive polymer is applied to the base material M after heating the base material M or while heating the base material M, and subsequently the producing solution containing the oxidizing agent and the dopant are applied, and therefore such operation can immediately perform the chemical polymerization reaction to minimize the bleeding. Moreover, the viscosity improver is added to the producing solution, and therefore such operation can prevent the producing solution from spreading upon applying the producing solution to the base material M to minimize the bleeding. Moreover, addition of the viscosity improver simultaneously causes action of reducing a resistance value to a half or less. Accordingly, the conductive polymer can be adhered to the base material M in the objective shape with high accuracy and with high electrical conductivity.

Moreover, if the unreacted substances in the raw material solution and the producing solution applied to the base material M are washed and removed, such operation can prevent the unreacted substances from gradually reacting to cause spreading of the bleeding.

Further, if the raw material solution and the producing solution are applied to the base material M by moving the raw material application means 12 for applying the raw material solution thereto and the producing solution application means 13 for applying the producing solution thereto relative to the base material M, such operation can easily adhere the conductive polymer to the base material M in the objective shape.

In addition thereto, if the base material M is partially heated by moving the position on which the base material M is heated in corresponding to the positions of the raw material application means 12 and the producing solution application means 13 relative to the base material M, such operation can easily control the reaction time to minimize the resistance of the conductive polymer conductor.

Furthermore, if the fixing material for fixing the conductive polymer to the base material M is applied to the base material M, such operation can adhere the conductive polymer thereto, irrespective of a material of the base material M.

The device 10 for producing the conductive polymer conductor according to the embodiment is equipped with the heating means 11 for heating the base material M, the raw material application means 12 for applying the raw material solution to the base material M, and the producing solution application means 13 for applying the producing solution to the base material M, and therefore such configuration can easily realize the method for producing the conductive polymer conductor in the present embodiment.

### Industrial Applicability

The method can be applied to a conductive polymer conductor in which a conductive polymer is adhered to a base material.

### Reference Signs List

10 ... Device for producing conductive polymer conductor, 11 ... heating means, 12 ... raw material application means, 13 ... producing solution application means, 14 ... application position moving means, 15 ... heating position control means.

## Claims

1. A method for producing a conductive polymer conductor in which a conductive polymer is adhered to a base material (M), comprising:
a raw material application step of applying, to the base material (M), a raw material solution containing a monomer of the conductive polymer after heating the base material (M) or while heating the base material (M); and
a producing solution application step of applying, to the base material (M) being in a heated state, a producing solution containing an oxidizing agent for promoting polymerization of the monomer, a dopant for developing electrical conductivity in the conductive polymer and a viscosity improver for improving viscosity after applying the raw material solution to the base material (M).

2. The method for producing the conductive polymer conductor according to claim 1, comprising:
a washing step of washing and removing unreacted substances in the raw material solution and the producing solution applied after applying the raw material solution and the producing solution to the base material (M).

3. The method for producing the conductive polymer conductor according to claim 1 or 2, wherein the raw material solution and the producing solution are applied to the base material (M) by moving the raw material application means (12) for applying the raw material solution thereto, and the producing solution application means (13) for applying the producing solution thereto relative to the base material (M).

4. The method for producing the conductive polymer conductor according to claim 3, wherein the base material (M) is partially heated by moving a position on which the base material (M) is heated in corresponding to positions of the raw material application means (12) and the producing solution application means (13) relative to the base material (M).

5. The method for producing the conductive polymer conductor according to any one of claims 1 to 4, wherein sericin is applied to the base material (M).

6. The method for producing the conductive polymer conductor according to any one of claims 1 to 5, comprising a fixing material application step of applying, to the base material (M), a fixing material for fixing the conductive polymer to the base material (M) before applying the raw material solution to the base material (M).

7. The method for producing the conductive polymer conductor according to any one of claims 1 to 6, wherein the conductive polymer is poly3,4-ethylenedioxythiophene.

## Patentansprüche

1. Verfahren zum Herstellen eines leitfähigen Polymerleiters, in welchem ein leitfähiges Polymer an einem Grundmaterial (M) anhaftet, umfassend:
einen Rohmaterialauftragungsschritt des Auftragens, auf das Grundmaterial (M), einer ein Monomer des leitfähigen Polymers enthaltenden Rohmateriallösung nach Heizen des Grundmaterials (M) oder während Heizens des Grundmaterials (M); und
einen Herstellungslösungsauftragungsschritt des Auftragens, auf das Grundmaterial (M), das in einem geheizten Zustand ist, einer Herstellungslösung enthaltend ein Oxidationsmittel zum Fördern der Polymerisation des Monomers, einen Dotierstoff zum Entwickeln von elektrischer Leitfähigkeit in dem leitfähigen Polymer und einen Viskositätsverbesserer zum Verbessern der Viskosität nach Auftragen des Rohmaterials auf das Grundmaterial (M).

2. Verfahren zum Herstellen des leitfähigen Polymerleiters nach Anspruch 1, umfassend:
einen Waschschritt des Waschens und Entfernens nicht reagierter Substanzen in der Rohmateriallösung und der Herstellungslösung aufgebracht nach Auftragen der Rohmateriallösung und der Herstellungslösung auf das Grundmaterial (M).

3. Das Verfahren zum Herstellen des leitfähigen Polymerleiters nach Anspruch 1 oder 2, wobei die Rohmateriallösung und die Herstellungslösung auf das Grundmaterial (M) aufgebracht werden, indem die Rohmaterialauftragungsmittel (12) zum Auftragen der Rohmateriallösung darauf bewegt werden und das Auftragungsmittel für die Herstellungslösung (13) zum Auftragen der Herstellungslösung darauf relativ zu dem Grundmaterial (M).

4. Verfahren zum Herstellen des leitfähigen Polymerleiters nach Anspruch 3, wobei das Grundmaterial (M) teilweise erhitzt wird, indem eine Position bewegt wird, auf der das Grundmaterial (M) entsprechend den Positionen der Rohmaterialauftragungsmittel (12) und des Auftragungsmittels für die Herstellungslösung (13) relativ zum Grundmaterial (M) erhitzt wird.

5. Verfahren zum Herstellen des leitfähigen Polymerleiters nach einem der Ansprüche 1 bis 4, wobei Serizin auf das Grundmaterial (M) aufgebracht wird.

6. Verfahren zum Herstellen des leitfähigen Polymerleiters nach einem der Ansprüche 1 bis 5, umfassend einen Fixierungsmaterialauftragungsschritt des Auftragens, auf das Grundmaterial (M), eines Fixiermaterials zum Fixieren des leitfähigen Polymers auf dem Grundmaterial (M) vor Auftragen der Rohmateriallösung auf das Grundmaterial (M).

7. Verfahren zum Herstellen des leitfähigen Polymerleiters nach einem der Ansprüche 1 bis 6, wobei das leitfähige Polymer Poly-3,4-ethylendioxythiophen ist.

## Revendications

1. Procédé pour produire un conducteur polymère conducteur dans lequel un polymère conducteur adhère à un matériau de base (M), comprenant :
une étape d'application de matière première consistant à appliquer, au matériau de base (M), une solution de matière première contenant un monomère du polymère conducteur après le chauffage du matériau de base (M) ou pendant le chauffage du matériau de base (M) ; et
une étape d'application de solution de production consistant à appliquer, au matériau de base (M) qui est dans un état chauffé, une solution de production contenant un agent oxydant pour favoriser la polymérisation du monomère, un dopant pour développer une conductivité électrique dans le polymère conducteur et un agent améliorant la viscosité pour améliorer la viscosité après l'application de la solution de matière première au matériau de base (M).

2. Procédé pour produire un conducteur polymère conducteur selon la revendication 1, comprenant :
une étape de lavage consistant à laver et éliminer les substances n'ayant pas réagi dans la solution de matière première et la solution de production appliquée après l'application de la solution de matière première et de la solution de production au matériau de base (M).

3. Procédé pour produire un conducteur polymère conducteur selon la revendication 1 ou 2, dans lequel la solution de matière première et la solution de production sont appliquées au matériau de base (M) par déplacement des moyens d'application de matière première (12) pour y appliquer la solution de matière première, et des moyens d'application de solution de production (13) pour y appliquer la solution de production par rapport au matériau de base (M).

4. Procédé pour produire un conducteur polymère conducteur selon la revendication 3, dans lequel le matériau de base (M) est partiellement chauffé en étant déplacé à une position sur laquelle le matériau de base (M) est chauffé en correspondance avec les positions des moyens d'application de matière première (12) et des moyens d'application de solution de production (13) par rapport au matériau de base (M).

5. Procédé pour produire un conducteur polymère conducteur selon l'une quelconque des revendications 1 à 4, dans lequel de la séricine est appliquée au matériau de base (M).

6. Procédé pour produire un conducteur polymère conducteur selon l'une quelconque des revendications 1 à 5, comprenant une étape d'application de matériau de fixation consistant à appliquer, au matériau de base (M), un matériau de fixation pour fixer le polymère conducteur au matériau de base (M) avant l'application de la solution de matière première au matériau de base (M).

7. Procédé pour produire un conducteur polymère conducteur selon l'une quelconque des revendications 1 à 6, dans lequel le polymère conducteur est le poly(3,4-éthylènedioxythiophène).
